# EUROPEAN PATENT SPECIFICATION

(11) **EP 4 414 004 B1**
(45) Date of publication and mention of the grant of the patent: **07.10.2026**
(21) Application number: 24157453.2
(22) Date of filing: 13.02.2024
(51) Int. Cl.: A61M 1/36

(54) **BIOLOGICAL FLUID CONCENTRATION DEVICES AND METHODS OF USE**
VORRICHTUNG ZUR KONZENTRATION BIOLOGISCHER FLÜSSIGKEITEN UND VERFAHREN ZUR VERWENDUNG
DISPOSITIFS DE CONCENTRATION DE FLUIDE BIOLOGIQUE ET PROCÉDÉS D'UTILISATION

(30) Priority: 13.02.2023 US 202363484628 P
(43) Date of publication of application: 14.08.2024
(73) Proprietor: Arthrex, Inc., Naples, FL 34108 (US)
(72) Inventor: CHARLES, Joshua, Naples (US); BROUWER, Haley, Naples (US); PAWLYSHYN, Brianna, Naples (US); BARE, Christopher, Naples (US)
(74) Representative: Mathys & Squire

(56) References cited:
- EP-B1- 1 848 473
- US-A1- 2014 274 895

## Description

### Priority

This application claims the benefit of U.S. Ser. No. 63/484,628 filed on February 13, 2023.

### Background

Autologous fluid is commonly used in several areas of medicine, such as orthopedics. The addition of the patient's own proteins (e.g., growth factors), back into a site of injury of the healing process can be initiated or accelerated. A higher dose of these proteins is desirable. Other than increasing the volume of autologous fluid, a higher protein dose can be achieved by concentrating the proteins already present in the autologous fluid. Methods and devices are needed for concentration of proteins in not only in autologous fluids, but also allogenic fluids. US 2014/274895 A1 describes a device for concentrating biological fluids.

### Summary

An aspect provides a biological fluid concentration device. The device can comprise an upper body comprising a distal end and a proximal end, a chamber, and a valve (e.g., a swabbable slip tip type valve); a lower body comprising a distal end, a proximal end, a chamber containing a concentration material; a valve (e.g., a disk valve) at the distal end of the lower body; and a port such as a 90 degree port for mixing; and a filter, grate, or combination of a filter and a grate located between the upper body and the lower body; configured such that the biological fluid can flow from the lower chamber, through the filter or grate, and into the upper chamber. The lower body can further comprise threads at the distal end of the lower body. The lower body can further contain one or more ball bearings or agitator particles. The concentration material can be polyacrylamide beads. The lower body, upper body, and filter, grate, or combined filter and grate can be screwed together or press fit together. The filter, grate, or combination of filter and grate can have pores sized to retain the concentration material within the chamber of the lower device.

An aspect provides a method of concentrating a biological fluid. The method can comprise inserting a biological fluid into the chamber of the lower body of the biological fluid concentration device, shaking the device, inverting the device so that the lower body is orientated upwards and placing the device into a centrifuge, centrifuging the device such that concentrated biological fluid is collected in the upper body, and withdrawing the concentrated biological from the upper body. The lower body of the biological fluid concentration device can comprise threads at the distal end of the lower body. The biological fluid can be plasma, whole blood, serum, platelet rich plasma, buffy coat, platelet poor plasma, bone marrow mononuclear cells (BMMC), or stromal vascular fraction (SVF) from adipose tissue. The biological fluid can be inserted into the chamber of the lower body of the device by screwing a device for preparation of platelet rich plasma (PRP) onto the threads at the distal end of the lower body and directly inserting the PRP into the chamber of the lower body through the valve (e.g., the disk valve). A needle can be attached to the valve (e.g., a swabbable slip tip type valve) of the upper body. The concentrated biologic fluid of the chamber of the upper body can be injected into a ligament, tendon, bone, cartilage, or joint space of a patient, or can be prepared for such an injection.

Herein described but not part of the invention is a method of treatment of soft tissue damage or injury comprising isolating plasma from a patient with soft tissue damage or injury and placing the plasma in the chamber of the lower body of a device as described herein, shaking the device; inverting the device so that the lower body is orientated upwards and placing the device into a centrifuge; centrifuging the device such that concentrated plasma is collected in the upper body; withdrawing concentrated plasma from the upper body; and contacting the soft tissue damage or injury with the concentrated plasma. Also described is a patient's concentrated plasma for use in a method of treating soft tissue damage or injury by contacting the patient's soft tissue damage or injury with the concentrated plasma, wherein the plasma has been concentrated by a method comprising: placing plasma which has been isolated from a patient with soft tissue damage or injury in the chamber of the lower body of a device as described herein, shaking the device; inverting the device so that the lower body is orientated upwards and placing the device into a centrifuge; centrifuging the device such that concentrated plasma is collected in the upper body; and withdrawing the concentrated plasma from the upper body. The soft tissue damage or injury can be damage or injury to a ligament, tendon, muscle, or joint. The tendon can be a patellar tendon, anterior tibialis tendon, Achilles tendon, Hamstring tendon, semitendinosus tendon, gracilis tendon, abductor tendon, adductor tendon, supraspinatus tendon, infraspinatus tendon, subscapularis tendon, teres minor tendon, flexor tendon, rectus femoris tendon, tibialis posterior tendon, or quadriceps femoris tendon. The ligament can be an anterior cruciate ligament, lateral collateral ligament, posterior cruciate ligament, medial collateral ligament, cranial cruciate ligament, caudal cruciate ligament, cricothyroid ligament, periodontal ligament, suspensory ligament of the lens, anterior sacroiliac ligament, posterior sacroiliac ligament, sacrotuberous ligament, sacrospinous ligament, inferior pubic ligament, superior pubic ligament, suspensory ligament, palmar radiocarpal ligament, dorsal radiocarpal ligament, ulnar collateral ligament, or radial collateral ligament. The soft tissue injury or damage can be tendon or ligament rupture, severance, tearing, delamination, strain, or deformation.

Yet another aspect provides a kit comprising a biological fluid concentration device as described herein, and one or more of an injection kit, a needle, a syringe, a buffer, a connector, tubing, a luer lock, a top vented cap, a bottom vented cap, a top non-vented cap, a bottom non-vented cap, a top plug, and a bottom plug.

### Brief Description of Figures

Fig. 1 shows an example of a biological fluid concentrator.

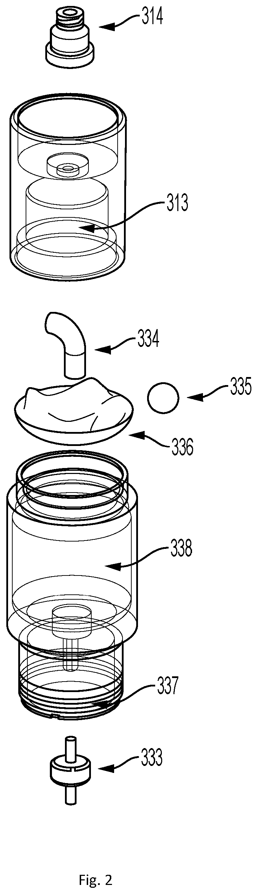

Fig. 2 shows an example of a biological fluid concentrator.
Fig. 3 shows an example of a use of a biological fluid concentrator.
Fig. 4A-4C shows an example of a device for obtaining platelet rich plasma.
Fig. 5A-B shows an example of a device for obtaining platelet rich plasma.
Fig. 6 shows an example of a filter and grate combination.

### Detailed Description

Biological fluid concentration devices described herein can provide increased concentrations of proteins in biological fluids such as autologous and allogenic fluids while minimizing sterility exposures by transferring fluids via luer locks and tubes. Additionally, the biological fluid concentration devices can directly attach to platelet rich plasma (PRP) concentration devices.

### Biological Fluid Concentration Devices

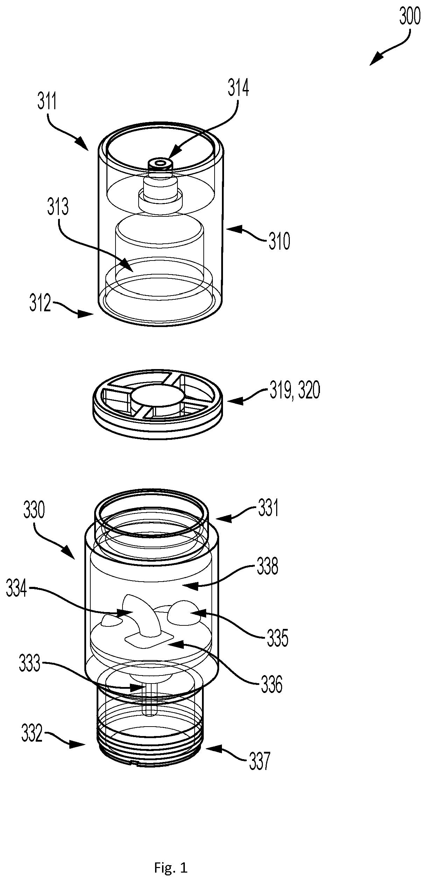

Biological fluid concentration devices can be used to, for example, concentrate proteins in a biological fluid such as autologous or allogenic plasma or autologous or allogenic serum. A biological fluid concentration device **300** can comprise an upper body **310,** a lower body **330,** and a filter **319** or grate **320** located between the upper body and the lower body. See Fig. 1.

An upper body **310** can comprise a distal end **312** and a proximal end **311,** a chamber **313,** and a swabbable slip tip type valve **314.** A slip tip type valve can be a slip tip, luer-slip or luer-lock style port (e.g., a port having a friction-fit connector). The valve **314** can comprise a filter. The lower body **330** can comprise a distal end **332,** a proximal end **331,** a chamber **338** containing, in some aspects, one or more ball bearings or agitator particles **335** and a concentrating material such as polyacrylamide beads **336;** a valve such as a disk valve **333** at the distal end **332** of the lower body **330;** and a port such as a 90 degree port **334.** An upper body and lower body can be about 0.5, 0.7, 1.0, 1.2, 1 .5, 2.0, 3.0, 4.0, 5.0, 6.0, 7.0, 8.0 centimeters or more in diameter.

### Concentrating Material

A concentrating material can be a continuous or discontinuous material that can reduce the volume of liquid of a biological sample (e.g., concentrating the elements contained within the biological sample). For example, the concentrating material can comprise porous beads. The concentrating material can comprise an absorbent material, such as desiccating polyacrylamide beads, which absorb a portion of the biological sample liquid. In an embodiment, a concentrating material comprises porous glass beads, dextranomer beads, hydrous SEPHADEX ^{™} water absorbers, silica gel, zeolite, crosslinked agarose, gels, wool, powder, plastic, granules, fibers, porous material or combinations thereof that can absorb liquid contained the biological sample. Various examples of suitable concentration materials include dry hydrogel beads, glasses, minerals such as corundum and quartz, polymers, and polysaccharides. Polymers include, for example, polystyrene, polyethylene, polyvinyl chloride, polypropylene, and polyacrylamide. Polysaccharides include, for example, dextran and agarose. In an aspect, a concentrating material does not introduce any desired contaminants into the plasma.

The concentrating material can be about 0.01, 0.1, 0.5, 1.0, 2.0, 3.0, 4.0, 5.0 mm or more in size or diameter. The diameter or size of the concentrating material can increase with the volume of liquid absorbed by the concentrating material. The concentrating material can comprise a continuous porous network, similar to a sponge, or can include a plurality of individual porous particles. The concentrating material can provide a larger surface area by being porous in comparison to a non-porous material. In some embodiments, the pores are too small for cells, e.g., blood cells (about 7-8 µm), to enter, but a portion of the liquid sample can enter the pores. In an example where the sample comprises blood cells the pores would be less than about 7 µm (e.g., about 7, 6, 5, 4, 3, 2, 1 µm or less).

### Filter and Grate

One or more filters, grates, or filters combined with grates can be present between the upper body and the lower body such that, when the upper body, lower body, filter and/or grate are assembled, the biological fluid can flow from the lower chamber, through the filter or filter and grate, and into the upper chamber. The pore size of the filter, grate, or filter combined with a grate is selected to prevent the concentrating material and ball bearings or agitator particles from passing through the filter and/or grate and into the upper body.

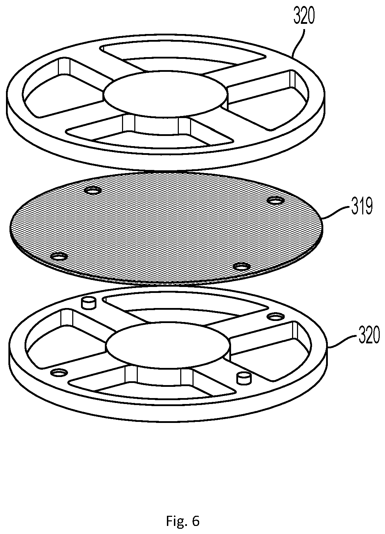

In an aspect, a filter and grate combination can comprise a filter sandwiched between two grates. See Fig. 6. In this aspect, the filter can prevent the concentrating material from entering the upper chamber, while the grates can prevent the ball bearings or agitator particles from entering the upper chamber and can protect the filter from damage from the ball bearings or agitator particles during shaking. A filter can be glued or ultrasonically welded to the grates.

A filter, grate, or combination of a grate and filter can have any shape and can be threaded to screw onto the upper and lower bodies or can be snap fit or press fit to the upper and lower bodies. A filter and/or grate can be about 0.5, 0.7, 1.0, 1.2, 1 .5, 2.0, 3.0, 4.0, 5.0, 6.0, 7.0, 8.0 centimeters or more in diameter.

A filter and/or grate can comprise a filter material, a solid perforated material, or a combination of a filter material and solid perforated material. The solid perforated material can include polystyrene or another solid, nonabsorbent material, and can be partially or fully translucent for visibility. The solid material can be perforated, that is, it can comprise a large number of small holes to provide an effective flow communication between the chamber of the lower body and the chamber of the upper body without allowing concentrating materials, beads, and/or ball bearings/agitator particles to pass through the holes. The filter material can include a membrane or a mesh having pores, which can have varying size. The solid perforated material or filter material can comprise for example, metals or plastics, like titanium, stainless steel, polyethylene, polytetrafluoroethylene PTFE, polyvinylidene fluoride (PVDF), nylon, polypropylene (PP), polyester, polycarbonate, polyethersulfone, cellulose acetate, polyimide, or another equivalent material. A filter or solid perforated material can have a pore size or hole size from about 5, 10, 25, 50, 100, 150, 200, 300 µm or more.

A filter, grate, or solid perforated material can be configured to retain beads, concentration materials, and ball bearings/agitator particles inside the lower chamber. That is, a filter, grate, or solid perforated material can comprise pores or holes having a size compatible with the size of the concentrating material and ball bearings/agitator particles. For example, if the concentrating materials and ball bearings/agitator particles are about 200 microns, the filter, grate, or perforated solid material can be designed to have about e.g., 150 micron pores, so that concentrating material and ball bearings/agitator particles having a size greater than about 150 microns can be retained in the lower chamber.

### Valve and Port

The lower body has a valve, such as a disk valve (Halkey Roberts, St. Petersburg, FL) or other one-way valve. A disk valve can be a one-way valve having a disc shaped valve body enclosed about the circumferential edge by an elastomeric membrane.

The valve is followed by a port that can disrupt or change the inflow of fluid for proper mixing, such as a 90-degree port, a T-shaped port, or other suitable port to allow for the introduction of fluid into the chamber of the lower body. The port can change the inflow of the fluid into the chamber so that it does not flow through the filter without proper mixing. The port can cause the fluid to be directed to the inner diameter of the chamber. The fluid can be introduced into the lower chamber using, for example, a female-female luer lock adapter (slip-tip connector) to a syringe, or a syringe or device for the preparation of PRP such as an ACP^{®} Max device can be directly connected to the valve. The lower body 330 can further comprises threads 337 at the distal end 332 of the lower body to connect, for example, a PRP concentrator, such as an ACP Max^{®} PRP concentrator.

Any of the valve and port can further comprise any type of filter.

### Biological Fluid Concentration Device

A biological fluid concentration device can be made of any suitable material. Any component or element of a biological fluid concentration device, for example, the upper and lower bodies, can be made of polycarbonate, polypropylene, or polyethylene, polystyrene, polyethylene terephthalate, acrylonitrile butadiene styrene, acetal, or polysulfone.

A biological fluid concentration device can comprise an upper body 310 comprising a distal end 312 and a proximal end 311, a chamber 313, and a swabbable slip tip type valve 314. The device can also comprise a lower body 330 comprising a distal end 332, a proximal end 331, and a chamber 338 containing a concentration material 336. The upper and lower bodies can have a round, oval or other suitable cross-section. The upper and lower bodies can have a diameter of about 0.5, 0.75, 1.0, 1.25, 1.5, 1.75, 2.0, 2.25, 2.5, 3.0 inches or more. The diameter can vary along the length of the bodies. The upper and lower bodies can each be about 1.0, 1.5, 1.75, 2.0, 2.25, 2.5, 2.75, 3.0, 3.25, 3.5, 3.75, 4.0, 4.25, 4.5, 4.75, 5.0 inches or more in length. When assembled the upper and lower bodies together can be about 1.0, 1.5, 2.0, 2.5, 3.0, 3.5, 4.0, 4.5, 5.0, 5.5, 6.0, 6.5, 7.0, 7.5, 8.5, 9.0, 9.5, 10.0 inches or more. The upper and lower body chambers can each hold about 3, 5, 10, 20, 30, 40, 50, 100 mL or more of fluid. The proximal end of the upper body can be sealed with a plug, vented cap, or unvented cap. The valve of the distal end of the lower body can be sealed with a plug, vented cap, or unvented cap. Any of the proximal end of the upper body, the distal end of the lower body, the vented or unvented caps can comprise any type of filter.

### Devices for Preparation of PRP (ACP^{®} MAX)

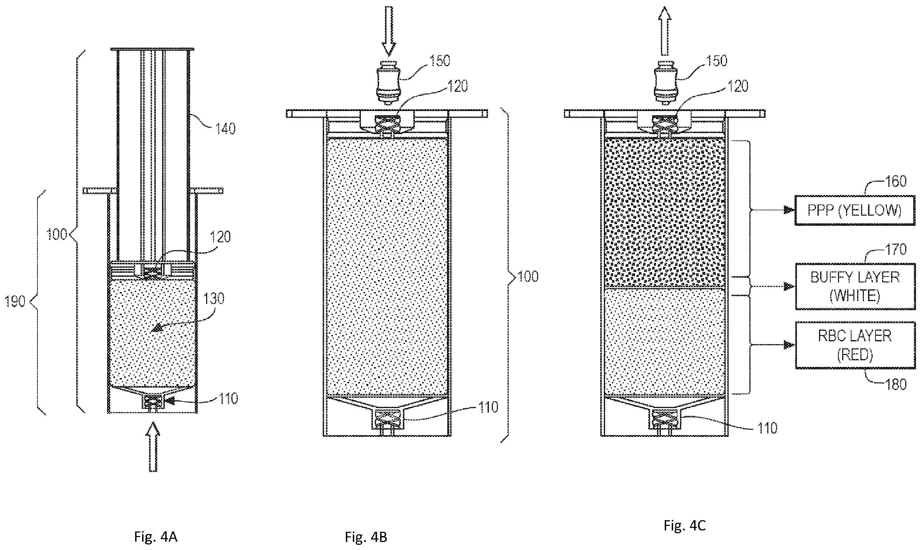

In some aspects, a biological fluid can be platelet rich plasma (PRP). PRP can be obtained using, for example, ACP^{®} MAX, a device for preparation of PRP. In an aspect, a device for preparation of PRP can comprise a first syringe 100 having a bottom coupling element 110, for, e.g. direct attachment of an infusion set to draw blood directly into the first syringe. FIG. 4A. A coupling element can be a luer lock connector or any other type of small-scale fluid fitting for making leak-free connections between a male-taper fitting and its mating female part. The first syringe also can comprise a top coupling element **120.** The top coupling element can be connected to, for example, a swabbable, needless injection luer connector **150** or a plunger **140.** The first syringe can comprise a barrel **190** with a chamber **130** suitable for biological fluids. Biological fluid can be drawn up into the chamber **130** of the first syringe with a plunger **140** connected to the top coupling element **120** (or other suitable means).

A biological fluid, e.g., whole blood, plasma, or serum can be drawn into the first syringe, either directly from the patient or from a container containing a previously collected sample of biological fluid. Any amount of biological fluid can be used, for example, about 10, 20, 50, 75, 100, 125, 150, 200, 300 or more mL. The plunger **140** can be removed from the top coupling element **120** and a swabbable needleless injector luer **150** (or other luer lock port or injector port) can be placed into the top coupling element (FIG. 4B). The first syringe can be placed into a centrifuge and spun under, for example, hard spin conditions. Three layers of biological fluids can be present after centrifugation: a platelet poor plasma (PPP) layer **160,** a buffy layer **170,** and a red blood cell or erythrocyte layer **180** (FIG. 4C).

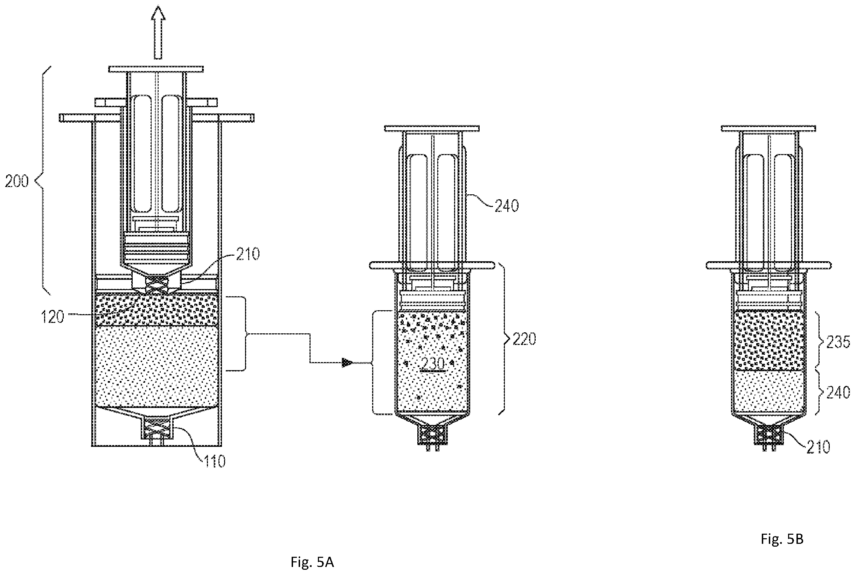

Optionally, an amount of the PPP layer (e.g., about 1, 2, 5, 10, 20, 30, 40, 50 mL or more) can be removed via the swabbable needless injector luer **150** or top coupling element **120** leaving behind the desired volume of PPP (e.g., about 50, 40, 30, 20, 10, 5, 2, 1, or less mL). The optionally removed PPP can be used for, e.g., dilution of the PRP final injection or to make an autologous clot. Alternatively, all of the PPP can be retained in the first syringe. A second syringe **200** or other second container can be connected to the top coupling element **120** or swabbable needless injector luer **150.** A second syringe can be an Arthrex ACP^{®} double syringe. A double syringe comprises, for example, a distal (outer) syringe having a first body with a first diameter; and a proximal (inner) syringe having a second body with a second diameter smaller than the first diameter, the distal (outer) syringe being in direct fluid communication with the proximal (inner) syringe, and the proximal (inner) syringe being located within the distal (outer) syringe. A second syringe or other second container can have a bottom coupling element **210** and a plunger **240.** The barrel **220** of the second syringe or second container can comprise a chamber **230** that can hold a biological fluid. The barrel of the second syringe or second container can have a smaller diameter than the first syringe so that the second syringe can fit within the barrel of the first syringe. See FIG. 5A.

All or part of the biological fluid present in the first syringe (e.g., PPP, buffy layer, red blood cell layer) can be drawn through the top coupling element **120** of the first syringe and into the second syringe **200** or other second container. FIG. 5A. In one example, the PPP layer, the buffy layer and optionally part of the red blood cell layer (e.g., about less than 1, 2, 5, 10, 15, or 30 mL) of the red blood cell layer can be drawn up into the second syringe or other second container. The second syringe or second container can be removed from the first syringe and placed into a centrifuge. The second syringe or second container can be centrifuged under, for example, soft spin conditions. Two layers can be present after the second centrifugation: a PRP layer **235** and an erythrocyte layer **240.** FIG. 5B. The PRP layer can be isolated by, for example, expelling the erythrocyte layer from the bottom coupling element **210** of the second syringe or second container. The PPP can then be used for direct injection into the patient through an injection set connected to bottom coupling element **210** of the second syringe or second container or can be stored in the second syringe or second container for later use, or removed from the chamber of the second syringe or second container and stored in another container for later use.

Where the second container is a double syringe, such as an Arthrex ACP^{®} double syringe, the plunger of the inner syringe can be used for easy isolation of PRP after the second centrifugation. The inner syringe (of the double syringe) can be pulled up to isolate the PRP after the second centrifugation. Therefore, the inner syringe of the second, double syringe can be used to pull up the PRP without disturbing the RBC layer. The inner syringe can then be directly connected to a biological fluid concentration device as described herein.

### Methods of Use of Biological Fluid Concentrator

Autologous or allogenic biological fluids such as blood, serum, plasma, PRP, platelet poor plasma, buffy coat layer, bone marrow mononuclear cells (BMMC), a stromal vascular fraction (SVF) of adipose tissue, or other enriched or concentrated biological fluids can be obtained using any suitable methods. The autologous or allogenic biological fluids can be *in vitro* or ex *vivo* samples. In some aspects a PRP concentrator, such as an ACP Max^{®} PRP concentrator, can be used to obtain PRP. In other aspects autologous or allogenic serum or plasma can be obtained using an Arthrex Angel^{®} System. Once obtained, the biological fluid can be inserted into the chamber of the lower body of the biological fluid concentrator. About 5, 10, 15, 20, 25, 30, 40, 50, 60, 70, 80, 90, 100 mL or more of biological fluid can be inserted into the chamber of the lower body. For example, An F-F luer lock can be used to insert the biological fluid into the chamber of the lower body of a biological fluid concentrator. In an aspect, biological fluid can inserted into the chamber of the lower body of the device by screwing a device for preparation of platelet rich plasma (PRP) onto the threads at the distal end of the lower body and directly inserting the PRP into the chamber of the lower body through the disk valve. A non-vented cap and/or a bottom plug can be used to close off the chamber of the lower body.

The biological fluid concentrator can be shaken vigorously for a suitable amount of time (e.g., 5, 10, 15, 20, 25, 30, 45, 60, or 120 seconds) until the concentrating material and biological fluid are well mixed in the chamber of the lower body. One or more (e.g., 1, 2, 3, 4, 5, 10 or more) ball bearings or agitator particles (e.g., glass, metal, plastic ball bearings or other suitable particles that can mix a sample when the sample is shaken or stirred) can be added to the chamber of the lower body to facilitate mixing in the chamber of the lower body. The biological fluid concentrator can be inverted and placed in a centrifuge and centrifuged for any suitable time to move the liquid in the chamber of the lower body through the filter and/or grate and into the chamber of the upper body. In some aspects, the device is centrifuged at about 500, 1,000, 1,500, 2,000, 2,500, or 3,000 RPM for 30 seconds, 1.0, 1.5, 2.0, 2.5, 3.0, 4.0 or more minutes. The cap of the upper body can be removed and the concentrated biological fluid can be withdrawn using any suitable methods. In an aspect, a primed syringe can be used to withdraw the concentrated biological fluid. Approximately 5, 10, 15, 20, 25 mL or more of biological fluid concentrate can be obtained. The concentrated fluid can be 0.1, 0.5, 1.0, 2.0, 5.0, or 10.0 fold more concentrated than the starting biological fluid. For example, the concentrated fluid can comprise a 1.0, 1.5, 1.75, 2.0, 3.0, 4.0, 5.0, 6.0, 7.0, 8.0, 9.0, 10.0, 11.0, 12.0, 13.0, 14.0, 15.0 fold or more increase of components such as IL-1ra, VEGF, FGF-b, A2M, TGF-b1, fibronectin, fibrinogen, total protein, platelets, red blood cells, and/or white blood cells when starting with, e.g., blood, serum, PPP or PRP or other suitable fluid as described herein.

### Methods of Use of Concentrated Biological Fluids

Herein described but not part of the invention is a composition comprising a concentrated biological fluid, e.g., plasma, for use in the treatment of soft tissue damage. In an aspect, where PPP is used, concentrated PPP can contain about 30, 40, 50, 60, 70, 80, 90 100 K/µL or more (thousands per cubic milliliter) platelets. In an aspect, where PRP is used, concentrated PRP can contain about 600, 750, 1,000, 1,250, 1,500, 1,750, 2,000, 2,250, 2,500, 2,750, 3,000 K/µL or more platelets. The concentrated biological fluid composition, e.g., plasma, can be derived by the methods described herein, and any features relating to concentrated biological fluid composition, e.g., plasma, produced by methods herein are applicable to the medical use thereof. The composition comprising an autologous concentrated biological fluid composition, e.g., plasma, can be for administration via injection into a ligament, tendon, bone, cartilage, or joint space.

About 0.1, 0.5, 1, 5, 10, 15, 20, 25 mL or more concentrated biological fluid composition, e.g., plasma, derived by the methods described herein can be administered to a subject. A subject can be a mammal. A mammal can be human, canine, feline, equine, or bovine. A subject can be a non-human mammal.

Concentrated biological fluid composition, e.g., plasma, derived by the methods described herein can be administered to a subject not undergoing surgery. A concentrated biological fluid composition, e.g., plasma, can be administered, such as by an injection, directly into cartilage, ligament, tendons, soft tissue, muscle, or a joint space of a subject not undergoing surgery. For example, a subject having chronic tendinopathies (e.g., Achilles tendinosis, lateral/medial epicondylitis, plantar fasciitis, patellar tendinopathy) can be administered concentrated biological fluid composition, e.g., plasma, via injection into a A subject having chondral injuries and early or advanced osteoarthritis can be administered a concentrated biological fluid composition, e.g., plasma.

Concentrated biological fluid composition, e.g., plasma, derived by the methods described herein can be administered to a subject during surgery. A concentrated biological fluid composition, e.g., plasma, can be administered during surgery to repair soft tissue damage or injury, such as for a ligament (e.g., anterior cruciate ligament, medical collateral ligament, etc.), cartilage (e.g., meniscus, labrum, etc.), or tendon (e.g., Achilles tendon). A concentrated biological fluid composition, e.g., plasma, can also be administered during surgery to promote bone repair (e.g., patella, tibia, femur, humerus, etc.). A concentrated biological fluid composition, e.g., plasma, can also be used to treat ischemia, ischemic stroke, heart injury or damage, muscle injury or damage, osteoarthritis, rheumatoid arthritis, and other diseases. A concentrated biological fluid composition, e.g., plasma, can be administered directly to soft tissue or to the joint space (e.g., shoulder, knee, ankle, wrist, hip, etc.).

Concentrated biological fluid composition, e.g., plasma, derived by the methods described herein can be administered to a subject with a neuropathy (e.g., peripheral neuropathy).

In the methods described herein, a concentrated biological fluid composition administered to a subject can be an autologous concentrated plasma. In an embodiment, the concentrated biological fluid composition administered to a subject can be an allogeneic concentrated plasma composition.

A concentrated biological fluid composition, e.g., plasma, derived by the methods described herein can be administered in combination with one or more specific growth factors. Growth factors include, but are not limited to, platelet-derived growth factor (PDGF), fibroblast growth factor (FGF), TGF-β (transforming growth factor-β), epithelial growth factor (EGF), and vascular endothelial growth factor (VEGF). Growth factors can be added to the concentrated biological fluid composition, e.g., plasma, or administered separately simultaneously or sequentially at about 0.001, 0.01, 0.1, 1.0, 10, 20, 50,100 or more ng/mL.

A concentrated biological fluid composition, e.g., plasma, derived by the methods described herein can be administered in combination with bone marrow concentrate (BMC), bone marrow aspirate (BMA), or lipoaspirate. About 1, 2, 5, 10, 15, 20, 25 mL or more BMC, BMA, or lipoaspirate can be added to the concentrated biological fluid composition, e.g., plasma, or separately administered simultaneously or sequentially.

A concentrated biological fluid composition, e.g., plasma, derived by the methods described herein can be administered in combination with stem cells. For example, the concentrated biological fluid composition, e.g., plasma, can be administered with mesenchymal stem cells or bone marrow stem cells. About 0.1, 0.5, 1, 2, 5, 10, 15, 20, 25 mL or more mesenchymal stem cells or bone marrow stem cells can be added to the concentrated biological fluid composition, e.g., plasma, or separately administered simultaneously or sequentially.

A concentrated composition derived by the methods herein can be administered directly from the concentration device to the patient through an injection kit attached to the concentration device, e.g., at the swabbable slip tip type valve 314. For example, once connected to an injection kit, the concentrated plasma composition can be directly administered to a patient.

### Kits

A kit can comprise a concentration device as described herein and one or more of an injection kit, a needle, a syringe, a buffer, connectors, tubing, luer locks, top vented caps, bottom vented caps, top non-vented caps, bottom non-vented caps, top plug, and bottom plug.

The terms "buffy layer" or "buffy coat" refer to the middle white layer of white blood cells, platelets, and mesenchymal and/or hematopoietic cells between the plasma layer and the erythrocyte layer following density gradient centrifugation or under hard spin centrifugation conditions of the blood.

The term "platelet poor plasma (PPP)" refers to a top layer of plasma following density gradient centrifugation of the blood or under hard spin centrifugation conditions of the blood. The PPP layer occurs when the centrifugation is at enough force where the white blood cells and platelets fractionate to the buffy layer.

The terms "erythrocyte" and "red blood cell (RBC)" are interchangeable and refer to mature red blood cells.

The term "hard spin" refers to centrifugation conditions that cause erythrocytes to sediment and platelets to sediment in a layer immediately above the erythrocyte layer and below a platelet poor plasma layer. Typically, a hard spin is defined as having a relative centrifugal force (RCF) of about 1500×g to about 2000×g. The term "soft spin" refers to centrifugation conditions that cause erythrocytes to sediment while platelets remain in suspension. Typically, a soft spin is defined as having an RCF of about 20×g to about 200×g.

The compositions and methods are more particularly described below and the Examples set forth herein are intended as illustrative only, as numerous modifications and variations therein will be apparent to those skilled in the art. The terms used in the specification generally have their ordinary meanings in the art, within the context of the compositions and methods described herein, and in the specific context where each term is used. Some terms have been more specifically defined herein to provide additional guidance to the practitioner regarding the description of the compositions and methods.

As used herein, the term "and/or" includes any and all combinations of one or more of the associated listed items. As used in the description herein and throughout the claims that follow, the meaning of "a", "an", and "the" includes plural reference as well as the singular reference unless the context clearly dictates otherwise. The term "about" in association with a numerical value means that the value varies up or down by 5%. For example, for a value of about 100, means 95 to 105 (or any value between 95 and 105).
In each instance herein any of the terms "comprising," "consisting essentially of," and "consisting of" can be replaced with either of the other two terms, while retaining their ordinary meanings. The terms and expressions which have been employed are used as terms of description and is recognized that various modifications are possible. Thus, it should be understood that although the present methods and compositions have been specifically disclosed by embodiments and optional features, modifications and variations of the concepts herein disclosed can be resorted to by those skilled in the art, and that the invention is defined by the appended claims.

Any single term, single element, single phrase, group of terms, group of phrases, or group of elements described herein can each be specifically excluded from the claims.

Whenever a range is given in the specification, for example, a temperature range, a time range, a composition, or concentration range, all intermediate ranges and subranges, as well as all individual values included in the ranges given are intended to be included in the disclosure. It will be understood that any subranges or individual values in a range or subrange that are included in the description herein can be excluded from the aspects herein. It will be understood that any elements or steps that are included in the description herein can be excluded from the claimed compositions or methods.

In addition, where features or aspects of the compositions and methods are described in terms of Markush groups or other grouping of alternatives, those skilled in the art will recognize that the compositions and methods are also thereby described in terms of any individual member or subgroup of members of the Markush group or other group.

The following examples are provided for exemplification purposes only.

### Examples

### Example 1

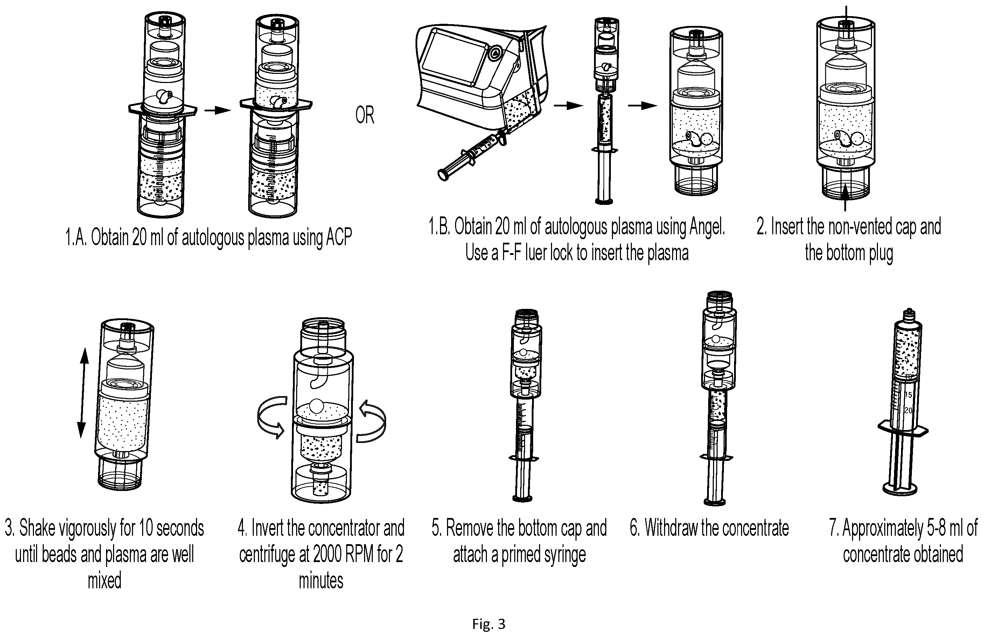

20 ml of autologous serum was obtained using ACP Max^{®} PRP concentrator. In other tests 20 ml of autologous serum was obtained using an Arthrex Angel^{®} System. An F-F luer lock was used to insert the plasma/serum into the lower chamber of a biological fluid concentrator. See Figure 3. A non-vented cap and bottom plug was used to close off the bottom chamber. The biological fluid concentrator was shaken vigorously for 10 seconds until 3.75 mg of polyacrylamide beads, 440C medical-grade stainless steel ball bearing, and plasma was well mixed. The biological fluid concentrator was inverted 180 degrees and centrifuged at 2,000 RPM for 2 minutes in Drucker (Horizon 24 Flex AH) or Hettich (Rotofix 32A) centrifuges. A 51µm nylon filter trapped the water-saturated polyacrylamide beads of different shapes and sizes in the lower chamber during centrifugation. The plasma passed through the filter into the upper body and was withdrawn through the Qosina swabbable luer-type valve. The cap of the upper chamber was removed and the concentrated plasma was withdrawn using a primed syringe. Approximately 5-8 ml of concentrate was obtained.

### Example 2

Devices as described herein were used to concentrate PRP and PPP. See Table 1. The devices were compared to Zimmer Biomet's Plasmax^{®} Plasma concentration System. Baseline levels of various proteins were measured in the PPP/PRP as well as the concentrated versions of the PPP/PRP. These numbers were then used to calculate the fold change (FoldX) in the protein concentration.

**Table 1. Protein and cell concentration by plasma concentrating systems. Data shown as average ± standard deviation.**

| | | **Arthrex Device with PPP** | **Plasmax^{®} Device with PPP** | **Arthrex Device with PRP** |
|---|---|---|---|---|
| | Number of trials | 12 | 12 | 6 |

| **Protein and Growth Factors** | | | | |
|---|---|---|---|---|
| IL-1ra | Plasma | 249.9 ± 139.7 | 249.9 ± 139.7 | 1694.1 ± 1443.7 |
| | Concentrate | 625.0 ± 414.3 | 417.1 ± 272.8 | 12235.1 ± 8076.5 |
| | FoldX | **2.88 ± 2.19** | **1.70 ± 0.40** | **12.19 ± 11.52** |
| VEGF (pg/ml) | Plasma | NA | NA | 108.1 ± 49.3 |
| | Concentrate | NA | NA | 223.4 ± 178.9 |
| | FoldX | NA | NA | **1.74 ± 0.70** |
| PDGF-ab (pg/ml) | Plasma | 273.8 ± 132.5 | 273.8 ± 132.5 | 4343.3 ± 1734.8 |
| | Concentrate | 538.7 ± 285.5 | 479.5 ± 242.1 | 2531.1 ± 643.2 |
| | FoldX | **2.01 ± 0.78** | **1.79 ± 0.67** | **0.74 ± 0.18** |
| FGF-b (pg/ml) | Plasma | 2.5 ± 1.3 | 2.5 ± 1.3 | 103.1 ± 64.5 |
| | Concentrate | 5.9 ± 3.3 | 4.2 ± 2.5 | 116.9 ± 111.0 |
| | FoldX | **2.34 ± 0.59** | **1.69 ± 0.47** | **0.93 ± 0.42** |
| A2M (µg/ml) | Plasma | 955.5 ± 266.1 | 955.5 ± 266.1 | 986.3 ± 156.3 |
| | Concentrate | 2546.8 ± 536.5 | 1939.8 ± 703.1 | 1893.0 ± 586.0 |
| | FoldX | **2.74 ± 0.51** | **2.05 ± 0.47** | **1.80 ± 0.94** |
| TGF-b1 (ng/ml) | Plasma | 1.7 ± 0.4 | 1.7 ± 0.4 | 44.5 ± 13.7 |
| | Concentrate | 2.9 ± 1.0 | 2.4 ± 0.5 | 32.9 ± 12.0 |
| | FoldX | **1.68 ± 0.40** | **1.43 ± 0.26** | **0.87 ± 0.34** |
| Fibronectin (µg/ml) | Plasma | 99.7 ± 66.1 | 99.7 ± 66.1 | 111.7 ± 62.8 |
| | Concentrate | 625.1 ± 288.3 | 347.9 ± 199.9 | 468.5 ± 236.2 |
| | FoldX | **8.5 ± 6.1** | **4.4 ± 2.8** | **5.6 ± 6.1** |
| Fibrinogen | Plasma | 28.7 ± 4.3 | 28.7 ± 4.3 | 25.2 ± 3.8 |
| (mg/ml) | Concentrate | 60.4 ± 10.1 | 50.7 ± 8.1 | 45.8 ± 10.4 |
| | FoldX | **2.12 ± 0.34** | **1.80 ± 0.33** | **1.83 ± 0.37** |
| Total Protein (mg/ml) | Plasma | 66.6 ± 8.0 | 66.6 ± 8.0 | 63.7 ± 8.3 |
| | Concentrate | 166.8 ± 16.1 | 123.9 ± 23.6 | 159.4 ± 40.4 |
| | FoldX | **2.53 ± 0.34** | **1.87 ± 0.33** | **2.45 ± 0.53** |

| **Cellular Components** | | | | |
|---|---|---|---|---|
| PLT (k/µl) | Plasma | 21.3 ± 8.5 | 21.3 ± 8.5 | 1171.0 ± 150.3 |
| | Concentrate | 55.9 ± 21.8 | 37.3 ± 12.8 | 1104.3 ± 550.5 |
| | FoldX | **2.7 ± 0.5** | **1.8 ± 0.4** | **1.0 ± 0.4** |
| RBC (M/µl) | Plasma | 0.0 ± 0.0 | 0.0 ± 0.0 | 0.7 ± 0.1 |
| | Concentrate | 0.0 ± 0.0 | 0.0 ± 0.0 | 1.2 ± 0.3 |
| | FoldX | **1.1 ± 0.3** | **1.1 ± 0.3** | **1.8 ± 0.7** |
| WBC (k/µl) | Plasma | 0.1 ± 0.1 | 0.1 ± 0.1 | 14.9 ± 2.8 |
| | Concentrate | 0.1 ± 0.1 | 0.1 ± 0.1 | 23.5 ± 4.1 |
| | FoldX | **1.4 ± 0.4** | **1.6 ± 0.8** | **1.6 ± 0.3** |

When undiluted, the concentrations of IL-1β in the PPP, PRP, and plasma concentrate samples were negligible. Therefore, the fold X of IL-1β by the plasma concentration systems was not calculated. The baseline concentrations of VEGF in PPP were negligible, thus the fold X of VEGF in PPP samples was not calculated. Data are reported for PRP samples.

## Claims

1. A biological fluid concentration device **300** comprising:
(i) an upper body **310** comprising a distal end **312** and a proximal end **311,** a chamber **313,** and a valve **314;**
(ii) a lower body **330** comprising a distal end **332,** a proximal end **331,** a chamber **338** containing a concentration material **336;** a valve **333** at the distal end **332** of the lower body **330;** and a port **334;** and
(iii) a filter **319,** grate **320,** or combination of a filter and a grate located between the upper body and the lower body; configured such that the biological fluid can flow from the lower chamber, through the filter and/or grate, and into the upper chamber;
**characterized in that** the port is a port for mixing.

2. The biological fluid concentration device of claim 1, wherein the lower body **330** further comprises threads **337** at the distal end **332** of the lower body.

3. The biological fluid concentration device of claim 1 or claim 2, wherein the lower body **330** further contains one or more agitator particles **335.**

4. The biological fluid concentration device of any of any preceding claim, wherein the concentration material **336** is polyacrylamide beads.

5. The biological fluid concentration device of any preceding claim, wherein the lower body, upper body, and filter, grate, or combination of filter and grate can be screwed together or press fit together.

6. The biological fluid concentration device of any preceding claim, wherein the filter, grate, or combination of filter and grate has pores sized to retain the concentration material within the chamber of the lower device.

7. An *ex vivo* method of concentrating a biological fluid comprising:
inserting biological fluid into the chamber of the lower body of the biological fluid concentration device of any of claims 1 to 6;
shaking the device;
inverting the device so that the lower body is orientated upwards and placing the device into a centrifuge;
centrifuging the device such that concentrated biological fluid is collected in the upper body;
withdrawing the concentrated biological fluid from the upper body.

8. The method of claim 7, wherein the lower body of the biological fluid concentration device comprises threads at the distal end of the lower body.

9. The method of claim 7 or claim 8, wherein the biological fluid is plasma, whole blood, serum, platelet rich plasma, buffy coat, platelet poor plasma, bone marrow mononuclear cells (BMMC), or stromal vascular fraction (SVF) from adipose tissue.

10. The method of claim 8 or claim 9, wherein the biological fluid is inserted into the chamber of the lower body of the device by screwing a device for preparation of platelet rich plasma (PRP) onto the threads at the distal end of the lower body and directly inserting the PRP into the chamber of the lower body through the valve **333.**

11. The method of any of claims 7 to 10, further comprising attaching a fluid extraction device to the valve **314** of the upper body, wherein said fluid extraction device is not fluidly connected to a patient.

12. A kit comprising the biological fluid concentration device of any of claims 1 to 6, and one or more of an injection kit, a needle, a syringe, a buffer, a connector, tubing, a luer lock, a top vented cap, a bottom vented cap, a top non-vented cap, a bottom non-vented cap, a top plug, and a bottom plug.

## Patentansprüche

1. Vorrichtung **300** für die Konzentration biologischer Flüssigkeiten, die Folgendes umfasst:
(i) einen oberen Körper **310**, der ein distales Ende **312** und ein proximales Ende **311**, eine Kammer **313** und ein Ventil **314** umfasst;
(ii) einen unteren Körper **330**, der ein distales Ende **332**, ein proximales Ende **331**, eine Kammer **338**, die ein Konzentrationsmaterial **336** enthält; ein Ventil **333** an dem distalen Ende **332** des unteren Körpers **330**; und einen Anschluss **334**; und
(iii) ein Filter **319**, ein Gitter **320** oder eine Kombination aus einem Filter und einem Gitter, die sich zwischen dem oberen Körper und dem unteren Körper befindet; derart konfiguriert, dass die biologische Flüssigkeit aus der unteren Kammer durch das Filter und/oder das Gitter in die obere Kammer fließen kann;
**dadurch gekennzeichnet, dass** der Anschluss ein Anschluss für das Mischen ist.

2. Vorrichtung für die Konzentration biologischer Flüssigkeiten nach Anspruch 1, wobei der untere Körper **330** ferner Gewinde **337** an dem distalen Ende **332** des unteren Körpers umfasst.

3. Vorrichtung für die Konzentration biologischer Flüssigkeiten nach Anspruch 1 oder 2, wobei der untere Körper **330** ferner ein oder mehrere Rührerpartikel **335** enthält.

4. Vorrichtung für die Konzentration biologischer Flüssigkeiten nach einem der vorstehenden Ansprüche, wobei das Konzentrationsmaterial **336** Polyacrylamidkügelchen ist.

5. Vorrichtung für die Konzentration biologischer Flüssigkeiten nach einem der vorstehenden Ansprüche, wobei der untere Körper, der obere Körper und das Filter, das Gitter oder eine Kombination aus Filter und Gitter miteinander verschraubt oder durch Presspassung miteinander verbunden werden können.

6. Vorrichtung für die Konzentration biologischer Flüssigkeiten nach einem der vorstehenden Ansprüche, wobei das Filter, das Gitter oder die Kombination aus Filter und Gitter Poren aufweist, die derart bemessen sind, dass das Konzentrationsmaterial innerhalb der Kammer der unteren Vorrichtung zurückgehalten wird.

7. *Ex-vivo*-Verfahren für das Konzentrieren einer biologischen Flüssigkeit, das Folgendes umfasst:
Einsetzen von biologischer Flüssigkeit in die Kammer des unteren Körpers der Vorrichtung für die Konzentration biologischer Flüssigkeiten nach einem der Ansprüche 1 bis 6;
Schütteln der Vorrichtung;
Umdrehen der Vorrichtung, sodass der untere Körper nach oben ausgerichtet ist, und Platzieren der Vorrichtung in eine Zentrifuge;
Zentrifugieren der Vorrichtung, sodass die konzentrierte biologische Flüssigkeit in dem oberen Körper gesammelt wird;
Entnehmen der konzentrierten Körperflüssigkeit aus dem oberen Körper.

8. Verfahren nach Anspruch 7, wobei der untere Körper der Vorrichtung für die Konzentration biologischer Flüssigkeiten an dem distalen Ende des unteren Körpers Gewinde umfasst.

9. Verfahren nach Anspruch 7 oder 8, wobei die biologische Flüssigkeit Plasma, Vollblut, Serum, thrombozytenreiches Plasma, Buffy Coat, thrombozytenarmes Plasma, mononukleäre Knochenmarkszellen (BMMC) oder stromale vaskuläre Fraktion (SVF) aus Fettgewebe ist.

10. Verfahren nach Anspruch 8 oder 9, wobei die biologische Flüssigkeit in die Kammer des unteren Körpers der Vorrichtung eingebracht wird, indem eine Vorrichtung für die Herstellung von thrombozytenreichem Plasma (PRP) auf das Gewinde an dem distalen Ende des unteren Körpers geschraubt wird und das PRP direkt durch das Ventil **333** in die Kammer des unteren Körpers eingesetzt wird.

11. Verfahren nach einem der Ansprüche 7 bis 10, das ferner das Anbringen einer Flüssigkeitsentnahmevorrichtung an dem Ventil 314 des oberen Körpers umfasst, wobei die Flüssigkeitsentnahmevorrichtung nicht fluidisch mit einem Patienten verbunden ist.

12. Kit, das die Vorrichtung für die Konzentration biologischer Flüssigkeiten nach einem der Ansprüche 1 bis 6 umfasst und eines oder mehrere eines Injektionskits, einer Nadel, einer Spritze, eines Puffers, eines Verbinders, eines Schlauchs, eines Luer Locks, einer oben belüfteten Kappe, einer unten belüfteten Kappe, einer oben nicht belüfteten Kappe, einer unten nicht belüfteten Kappe, eines oberen Stopfens und eines unteren Stopfens.

## Revendications

1. Dispositif de concentration de liquide biologique **300** comprenant :
(i) un corps supérieur **310** comprenant une extrémité distale **312** et une extrémité proximale **311**, une chambre **313** et une vanne **314** ;
(ii) un corps inférieur **330** comprenant une extrémité distale **332**, une extrémité proximale **331**, une chambre **338** contenant un matériel de concentration **336** ; une vanne **333** au niveau de l'extrémité distale **332** du corps inférieur **330** ; et un orifice **334** ; et
(iii) un filtre 319, une grille 320, ou une combinaison d'un filtre et d'une grille situés entre le corps supérieur et le corps inférieur ; configurés de sorte que le liquide biologique peut s'écouler depuis la chambre inférieure, à travers le filtre et/ou la grille, et jusque dans la chambre supérieure ; **caractérisé en ce que** l'orifice est un orifice destiné au mélange.

2. Dispositif de concentration de liquide biologique selon la revendication 1, dans lequel le corps inférieur **330** comprend en outre des filets **337** au niveau de l'extrémité distale **332** du corps inférieur.

3. Dispositif de concentration de liquide biologique selon la revendication 1 ou la revendication 2, dans lequel le corps inférieur **330** contient en outre une ou plusieurs particules d'agitation **335**.

4. Dispositif de concentration de liquide biologique selon l'une quelconque des revendications précédentes, dans lequel le matériel de concentration **336** est en billes de polyacrylamide.

5. Dispositif de concentration de liquide biologique selon l'une quelconque des revendications précédentes, dans lequel le corps inférieur, le corps supérieur et le filtre, la grille ou la combinaison de filtre et de grille peuvent être vissés ensemble ou assemblés par pression.

6. Dispositif de concentration de liquide biologique selon l'une quelconque des revendications précédentes, dans lequel le filtre, la grille ou la combinaison de filtre et de grille a des pores dimensionnés pour retenir le matériel de concentration à l'intérieur de la chambre du dispositif inférieur.

7. Procédé *ex vivo* de concentration d'un liquide biologique comprenant :
l'insertion d'un liquide biologique dans la chambre du corps inférieur du dispositif de concentration de liquide biologique selon l'une quelconque des revendications 1 à 6 ;
le secouement du dispositif ;
l'inversion du dispositif de sorte que le corps inférieur est orienté vers le haut et le positionnement du dispositif dans une centrifugeuse ;
la centrifugation du dispositif de sorte que le liquide biologique concentré est recueilli dans le corps supérieur ;
le retrait du liquide biologique concentré du corps supérieur.

8. Procédé selon la revendication 7, dans lequel le corps inférieur du dispositif de concentration de liquide biologique comprend des filets au niveau de l'extrémité distale du corps inférieur.

9. Procédé selon la revendication 7 ou la revendication 8, dans lequel le liquide biologique est du plasma, du sang total, du sérum, du plasma riche en plaquettes, de la couche leucocyto-plaquettaire, du plasma pauvre en plaquettes, des cellules mononucléaires de la moelle osseuse (BMMC), ou une fraction vasculaire stromale (SVF) provenant de tissu adipeux.

10. Procédé selon la revendication 8 ou la revendication 9, dans lequel le liquide biologique est inséré dans la chambre du corps inférieur du dispositif en vissant un dispositif de préparation de plasma riche en plaquettes (PRP) sur les filets au niveau de l'extrémité distale du corps inférieur et en insérant directement le PRP dans la chambre du corps inférieur par l'intermédiaire de la vanne **333**.

11. Procédé selon l'une quelconque des revendications 7 à 10, comprenant en outre la fixation d'un dispositif d'extraction de liquide à la vanne **314** du corps supérieur, dans lequel ledit dispositif d'extraction de liquide n'est pas en communication fluidique avec un patient.

12. Kit comprenant le dispositif de concentration de liquide biologique selon l'une quelconque des revendications 1 à 6, et un ou plusieurs éléments parmi un kit d'injection, une aiguille, une seringue, un tampon, un connecteur, un tube, un embout Luer Lock, un capuchon ventilé supérieur, un capuchon ventilé inférieur, un capuchon non ventilé supérieur, un capuchon non ventilé inférieur, un bouchon supérieur et un bouchon inférieur.
